# EUROPÄISCHE PATENTSCHRIFT

(11) **EP 0 710 132 B1**
(45) Veröffentlichungstag und Bekanntmachung des Hinweises auf die Patenterteilung: **29.01.1997**
(21) Anmeldenummer: 93922950.6
(22) Anmeldetag: 13.10.1993
(51) Int. Cl.: A61M 25/00

(54) **MANSCHETTENKATHETER**
SLEEVE CATHETHER
CATHETER A MANCHON

(30) Priorität: 19.07.1993 DE 4324218
(43) Veröffentlichungstag der Anmeldung: 08.05.1996
(73) Patentinhaber: Bavaria Medizin Technologie GmbH, D-82234 Wessling/Oberpfaffenhofen (DE)
(72) Erfinder: HÖFLING, Berthold, D-82234 Wessling (DE)
(74) Vertreter: Fleuchaus, Leo, Dipl.-Ing.
(86) Internationale Anmeldenummer: EP9302830
(87) Internationale Veröffentlichungsnummer: WO9503081

(56) Entgegenhaltungen:
- US-A- 3 593 713
- US-A- 3 981 299
- US-A- 5 178 611
- US-A- 5 180 366

## Beschreibung

Die Erfindung betrifft einen Manschettenkatheter zur Benetzung und Infiltration einer Gefäßwand mit einem Fluid, insbesondere einem Arzneimittel, vorzugsweise während der routinemäßigen Behandlung mit einem Ballonkatheter.

Für die Behandlung von Gefäßverengungen in Arterien ist die Ballondilatation als wenig invasive, sehr wirkungsvolle und elegante Methode anerkannt. Die Erfahrung lehrt jedoch, daß die Behandlungsmethode eine hohe Rezidivrate in der Größenordnung von etwa 30% der behandelten Patienten hat, d.h. die Gefäßverengung stellt sich innerhalb von mehreren Monaten, z. B. zwischen 3 und 6 Monaten wieder ein. Es gilt als nachgewiesen, daß dabei Zellwachstumsvorgänge eine Rolle spielen, die durch die Ballondilatation, d.h. die Aufweitung und das Reißen des Gewebes ausgelöst werden. Es ist daher wünschenswert, in dem der Ballondilatation unterworfenen Gefäßbereich eine Zusatzbehandlung durchzuführen, die einem solchen Gewebewachstum entgegenwirkt.

Es sind unterschiedliche Methoden für wachstumshemmende Eingriffe bekannt, insbesondere durch Energieapplikation, z.B. mit einem Laser, oder durch lokale Medikamentenverabreichung. Als Träger der lokalen Medikamentenverabreichung sind sog. Stents (Drahtwandstützen) bekannt, deren Einsatz jedoch umständlich ist, und die sich auch nicht entfernen lassen.

Es wurde auch schon vorgeschlagen Ballonkatheter zu benutzen, bei denen der Ballon perforiert ist, so daß während der Ballondilatation ein Arzneimittel appliziert werden kann, das in das Gewebe eindringen soll. Dieser Vorschlag erweist sich jedoch als unzweckmäßig, da bereits zu Beginn und während der Ballonentfaltung unkontrollierbare Mengen des Arzneimittels verloren gehen, so daß es nicht möglich ist, während der Behandlung eine definierte Arzneimittelmenge für die Benetzung des Gewebes und das Eindringen in die Gefäßwand in dem zu behandelnden Bereich der Gefäßwand zu verabreichen. Beim Aufblasen des Ballons, und zwar bevor sich der Ballon an der Gefäßwand anlegt, fließt nämlich bereits ein wesentlicher Anteil des Medikaments mit dem Ergebnis ab, daß die vorzeitig freigesetzte Arzneimittelmenge nicht an die zu behandelnden Gefäßschichten gelangt, oder daß eine unerwünscht hohe Dosierung erforderlich ist, um den lokal zu behandelnden Gefäßbereich ausreichend mit dem Arzneimittel zu beeinflussen. Ein weiterer Nachteil wird darin gesehen, daß in einem perforierten Ballon nicht genügend Druck aufgebaut werden kann, um das verengte Gefäß aufzuweiten.

Aus der US-A-5 180 366 ist ein Manschettenkatheter bekannt, bei dem die Manschette auf einen Ballon aufgeschoben ist. Die Manschette besitzt keine proximale Verbindung, durch welche ein Arzneimittel zugeführt werden kann. Die Applikation des Arzneimittels an die Gefäßwand erfolgt allein durch das Andrücken der mit Arzneimittel präparierten Manschette.

Der aus der US-A-3 593 713 bekannte Katheter weist einen Ballon auf, um ein Gefäßsegment abschließen zu können. In dieses Segment wird dann ein Arzneimittel über eine perforierte Manschette zugeführt, welche mit einem elastischen hülsenartigen Mantel überzogen ist. Zur Applikation wird der Mantel zurückgerollt.

Es ist wünschenswert die in Verbindung mit einem perforierten Ballon geschilderten Nachteile zu überwinden. Dabei soll die Effektivität und die Eleganz der Ballondilatation erhalten bleiben und während des gleichen Eingriffs ohne wesentlichen zusätzlichen Zeitaufwand eine medikamentöse Behandlung des Gewebes und der Gefäßwand ermöglicht werden. Es ist daher Aufgabe der Erfindung die etablierte Ballonkathetertechnik so zu erweitern, daß einerseits die hohe Rezidivrate durch gleichzeitige Arzneimittelapplikation herabgesetzt werden kann, wobei gewährleistet ist, daß das Arzneimittel in der gewünschten Dosierung im wesentlichen nur an die zu behandelnden Gewebeteile gelangt.

Diese Aufgabe wird ausgehend von dem eingangs erwähnten Manschettenkatheter erfindungsgemäß dadurch gelöst, daß der Manschettenkatheter einen Kranz von im Querschnitt um ein zentrales Lumen verteilte Außenlumina aufweist, die im Kopfsegment in Austrittsöffnungen enden, daß der Manschettenkatheter mit seinem zentralen Lumen auf einen Ballonkatheter aufschiebbar ist, und den Ballon zumindest teilweise übergreift, daß das Kopfsegment auf der Außenseite zwischen den Austrittsöffnungen mit in Längsrichtung verlaufenden Radialeinschnitten derart versehen ist, daß die nicht durchschnittene Restdicke ein Gelenk bildet, daß die Austrittsöffnungen perforiert sind und sich durch die Applikation des Ballondrucks öffnen, und daß das über die Außenlumina zugeführte bzw. eingefüllte Fluid mit Hilfe eines angelegten Drucks durch die Außenöffnungen in das Gefäß bzw. die Gefäßwand applizierbar ist.

Die Maßnahmen der Erfindung bieten den Vorteil, daß der Manschettenkatheter auf einen Teil des deflatierten Ballons eines Ballonkatheters aufschiebbar ist, so daß das niedrige Ballonprofil, das zum Eindringen in eine Gefäßverengung notwendig ist, nicht verschlechtert wird. Durch das Aufblasen des Ballons wird das Kopfsegment des Manschettenkatheters gegen das zu behandelnde Gewebe gedrückt, so daß im Stadium der Dilatation das gewünschte Arzneimittel zusätzlich durch die Außenlumina und die Austrittsöffungen unmittelbar in das zu behandelnde Gewebe eingepreßt werden kann und in das Gewebe eindringt. Damit können zur Unterdrückung des angeregten Zellwachstums im Behandlungsbereich sowohl zelltoxisch wirkende Substanzen als auch gentechnologisch designierte Medikamente unmittelbar in den zu behandelnden Gewebebereich injiziert werden.

Eine weitere Ausgestaltung der Erfindung sieht vor, daß die Austrittsöffnungen präformiert sind und sich durch die Applikation des Ballondrucks öffnen. Es ist auch vorgesehen, daß die Austrittsöffnungen präformiert sind und sich durch die Applikation eines zusätzlichen Drucks auf die Außenlumina öffnen.

Um die Aufweitung des Kopfsegments des Manschettenkatheters zu begünstigen, sieht eine weitere Ausgestaltung der Erfindung vor, daß das Kopfsegment auf der Außenseite zwischen den Austrittsöffnungen mit in Längsrichtung verlaufenden Radialeinschnitten versehen ist. Das Aufweiten kann weiter begünstigt werden, indem auch in der das zentrale Lumen begrenzenden Innenwand zwischen den Außenlumina in Längsrichtung verlaufende Radialschnitte vorhanden sind.

Für die möglichst lokale Verabreichung eines Medikaments im unmittelbaren Bereich der Gefäßverengung oder Stenose ist es auch zweckmäßig, daß die Präformation der Austrittsöffnungen derart ausgeführt ist, daß sich die Austrittsöffnungen mit ansteigendem Druck vom distalen Ende ausgehend zum proximalen Ende des Kopfsegmentes hin verlaufend öffnen.

Je nach dem Anwendungsort des Manschettenkatheters in Verbindung mit einem Ballonkatheter in Gefäßen oder Organen kann es wünschenswert sein, unterschiedliche Arzneimittelmengen für die Gewebebenetzung bzw. Infiltration in das Gewebe zu applizieren. Aus diesem Grund sieht die Erfindung vor, daß über den Umfang des Kopfsegmentes zwischen sechs bis zwanzig Außenlumina verteilt vorgesehen sind.

Die Vorteile und Merkmale der Erfindung ergeben sich auch aus der nachfolgenden Beschreibung eines Ausführungsbeispiels in Verbindung mit den Ansprüchen und der Zeichnung. Es zeigen:
- Fig. 1: das auf einen Ballonkatheter aufgeschobene Kopfsegment eines Manschettenkatheters;
- Fig. 2: einen Schnitt durch das vordere Ende des Ballonkatheters mit aufgeschobenem Kopfsegment;
- Fig. 3: einen Schnitt durch einen aufgeblasenen Ballon mit einem aufgeweiteten Kopfsegment des Manschettenkatheters;
- Fig. 4: einen Schnitt längs der Linen IV - IV der Fig. 1;
- Fig. 5: die Anschlußvorrichtungen zum Betätigen des Ballonkatheters und des Manschettenkatheters.

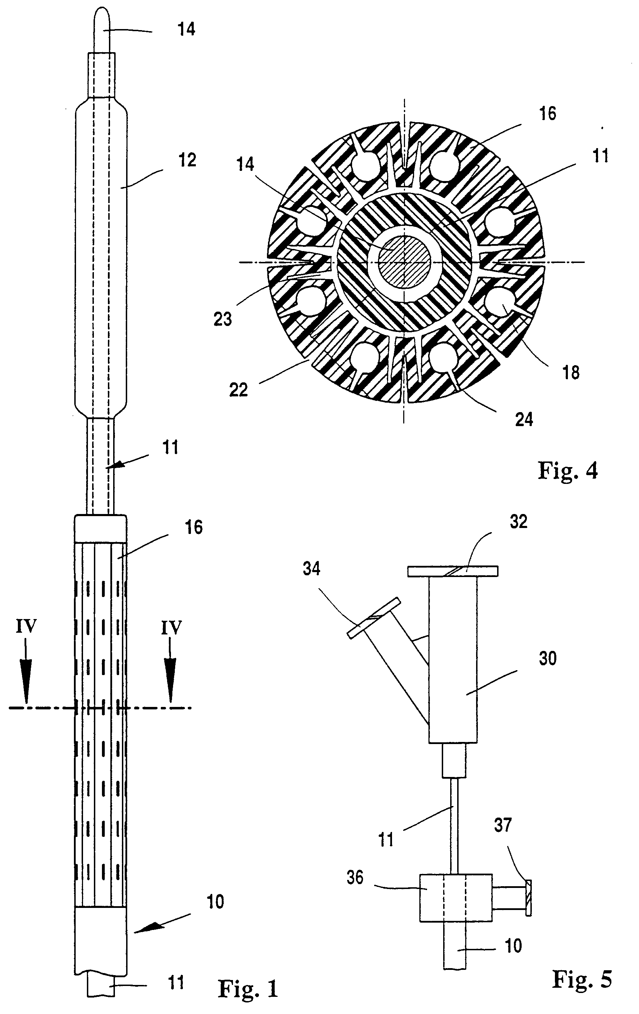

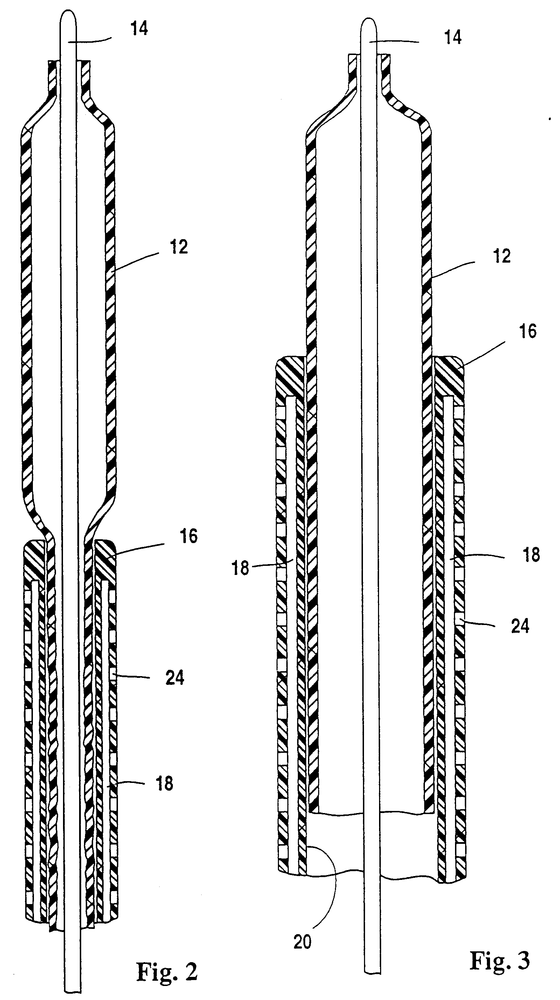

Aus den Fig. 1, 2 und 3 gehen Einzelheiten des auf einen Ballonkatheter 11 aufgeschobenen Manschettenkatheters 10 hervor. Der Manschettenkatheter ist auf den Ballonkatheter aufgeschoben, wobei das Kopfsegment 16 den Ballon 12 zumindest teilweise übergreift, wie aus Fig. 2 hervorgeht. In dieser Zuordnung werden die beiden Katheter in ein Gefäß oder Organ eingeführt. Im Kopfsegment des Manschettenkatheters enden die Außenlumina 18, welche sich über die gesamte Länge des Manschettenkatheters bis zu der in Fig. 5 dargestellten und später erläuterten Anschlußvorrichtung erstrecken. Es ist vorgesehen, daß diese Außenlumina 18 in einem Kranz um ein zentrales Lumen 20 angeordnet sind, wobei je nach Größe und Anwendungsgebiet des Manschettenkatheters zwischen 6 bis 20 Außenlumina 18 vorgesehen sein können.

Eine nicht dargestellte Ausführungsform sieht auch vor, daß in Längsrichtung des Manschettenkatheters nur ein Lumen bzw. 2 Lumina verlaufen, die sich im Kopfsegment in mehrere Außenlumina aufteilen können. Der Querschnitt und die Anordnung der Lumina wird an den jeweiligen Anwendungsfall angepaßt.

In dem in Fig. 4 dargestellten Schnitt durch das auf einem Ballonkatheter 11 aufgeschobene Kopfsegment 16 sind acht Außenlumina 18 vorgesehen, die Austrittsöffnungen 24 zur Außenseite des Kopfsegmentes haben. Für die Gestaltung dieser Austrittsöffnungen 24 ist vorgesehen, daß sie sich erst gegen Ende der Applikation des Ballondruckes öffnen und ein im Außenlumina 18 befindliches Medikament austreten lassen. Es ist auch vorgesehen, die Austrittsöffnungen präformiert so auszugestalten, daß sie sich durch die Applikation eines zusätzlichen über die Anschlußvorrichtung zugeführten Druckes öffnen.

Um das Aufweiten des Kopfsegmentes 16 zu erleichtern, ist dieses zumindest auf der Außenseite zwischen den Austrittsöffnungen 24 mit in Längsrichtung verlaufenden Radialeinschnitten 22 versehen, die nicht durch die Innenwand des Kopfsegmentes hindurchgehen. Dadurch entsteht ein Gelenk, aufgrund dessen sich der Radialeinschnitt 22 öffnen kann und die Aufweitung des Kopfsegmentes mit einem geringeren Druckaufwand möglich ist. Um den für die Aufweitung benötigten Druck weiter zu verringern, können weitere Radialeinschnitte 23 auf der das zentrale Lumen begrenzenden Innenseite des Kopfsegmentes vorgesehen sein, die in Längsrichtung zwischen den Außenlumina 18 verlaufen. Wenn beiderseits des Radialeinschnittes 22 zwei Radialeinschnitte 23 vorgesehen sind, ergeben sich drei Filmgelenke, die das Aufweiten des Kopfsegmentes weiter erleichtern.

Bei dieser Ausgestaltung kann das Kopfsegment mit dem für die vollständige Ausdehnung des Ballons benötigten Druck von beispielsweise 5x10⁵Pa bis 8x10⁵Pa (5 bis 8 bar) genügend fest an die Gefäßwand angedrückt werden, so daß bei der Applikation des Arzneimittels durch die Außenlumina 18 und die Austrittsöffnungen 24 dieses nicht abfließt, sondern mit genügend hoher Konzentration an die Wandinnenschicht gebracht und anschließend in tiefere Wandschichten mit genügend hoher Konzentration infiltriert werden kann.

Um die Verteilung des Arzneimittels im Stadium der Dilatation, wenn das Kopfsegment durch den im innenliegenden Ballon aufgebauten Druck gegen das gesprengte Gewebe gedrückt wird, möglichst im Zentrum der behandelten Engstelle injizieren zu können, ist vorgesehen, daß die Präformation der Austrittsöffnungen 24 derart ausgeführt ist, daß sich diese Austrittsöffnungen mit ansteigendem Druck vom distalen Ende ausgehend zum proximalen Ende des Kopfsegmentes hin verlaufend öffnen. Damit wird gewährleistet, daß das injizierte Arzneimittel nicht abfließt, sondern in genügend hoher Konzentration in die gewünschten Gewebeschichten und die anschließenden tieferen Wandschichten eingepreßt und infiltriert wird. Die nach der Katheterdeflation, welche in der Regel nach ca. 60 sek. stattfindet, eventuell noch im Gefäß freigesetzten Arzneimittelmengen sind dann verhältnismäßig gering und werden auf natürlichem Weg erdünnt, so daß sie für das Gesamtkörpersystem dosismäßig keine Rolle spielen, selbst wenn sie für den lokalen Gefäßabschnitt in hochdosierten Mengen appliziert werden. Für die Applikation kommen in erster Linie bereits wohlbekannte und in ihrer Wirkung gut abschätzbare zelltoxische Substanzen oder auch gentechnologisch designierte Medikamente in Frage.

Durch die Verwendung der üblichen bei der Ballondilatation benutzen Druckgeneratoren (Indeflator) können die Arzneimittel unter beliebig hohem Druck und in beliebiger, vorher festgelegter Menge in das Gewebe injiziert bzw. infiltriert werden.

In Fig. 5 ist die Anschlußvorrichtung für einen Indeflator sowie für eine Betätigungseinrichtung zum Einpressen eines Arzneimittels dargestellt. Diese Anschlußvorrichtung besteht aus einem Y-Konnektor mit einem Anschluß 32 für die Spülung und einem Anschluß 34 für den Indeflator. An diesen Y-Konnektor ist der Ballonkatheter 11 angeschlossen. Der auf den Ballonkatheter 11 aufgeschobene Manschettenkatheter 10 ist am hinteren Ende mit einem Konnektor 36 versehen, der einen Anschluß 37 hat, über welchen das Arzneimittel mit Hilfe eines nicht dargestellten Druckgenerators zugeführt werden kann. Innerhalb des Konnektors 36 ist der Anschluß 37 an die Außenlumina 18 des Manschettenkatheters angeschlossen.

Durch diese Ausgestaltung der Anschlußvorrichtung kann die Ballondilatation unabhängig von der Applikation des Arzneimittels erfolgen, und, wie bereits erwähnt, das Arzneimittel in beliebiger sowie festgelegter Menge verabreicht werden.

Bezogen auf die Behandlung von Herzkranzgefäßen liegt ein wesentlicher Vorteil der Erfindung darin, daß die Eleganz der Ballondilatationsmethode erhalten bleibt und der Eingriff nicht verlängert wird, weil die Injektion während der üblichen Ballondilatation erfolgt und eine genau definierbare Arzneimittelmenge in einem genau ersichtlichen Gefäßsegment appliziert werden kann.

Die Erfindung ist jedoch nicht nur für die Anwendung in Herzkranzgefäßen, sondern auch in Nierengefäßen, Extremitätengefäßen und gegebenenfalls auch in Kopfgefäßen geeignet. Sie kann auch für die Benetzung und Infiltration von Gefäßsegmenten mit wachstumshemmenden Substanzen in Organen oder röhrenförmigen Systemen Verwendung finden, wie z.B. der Harnröhre, der Gallengänge und anderen gefäßartigen Organen. Insbesondere für die Behandlung von krebserkrankten Bereichen mit wachstumshemmenden Substanzen (in diesem Fall Zytostatika) ist die Erfindung besonders gut geeignet. Damit wird die Behandlung von röhrenförmigen und von Karzinom befallenen Strukturen möglich, ohne den Transport durch diese Strukturen zu gefährden, d.h. auch bösartige Erkrankungen im Trachealraum oder im Darm können nach diesem Prinzip behandelt werden, ohne daß die Passage durch die erkrankte Struktur gestört wird. Auch für die Behandlung der Prostatahypertrophie können Medikamente auf die beschriebene Weise herangeführt werden.

## Patentansprüche

1. Manschettenkatheter zur Benetzung und Infiltration einer Gefäßwand oder anderen röhrenförmigen Organen mit einem Fluid, insbesondere einem Arzneimittel, mit einem als Manschette ausgebildeten, ein Arzneimittel abgebenden Kopfsegment, das sich durch Aufblasen eines Ballons (12) bis zur Anlage an der Innenwand des Gefäßes aufweiten läßt,
**dadurch gekennzeichnet,**
- daß der Manschettenkatheter (10) einen Kranz von im Querschnitt um ein zentrales Lumen (20) verteilten Außenlumina (18) aufweist, die im Kopfsegment (16) in Austrittsöffnungen (24) enden,
- daß der Manschettenkatheter (10) mit seinem zentralen Lumen (20) auf einen Ballonkatheter (11) aufschiebbar ist und den Ballon (12) zumindest teilweise übergreift,
- daß das Kopfsegment (16) auf der Außenseite zwischen den Austrittsöffnungen (24) mit in Längsrichtung verlaufenden Radialeinschnitten (22) derart versehen ist, daß die nicht durchschnittene Restdicke ein Gelenk bildet,
- daß die Austrittsöffnungen (24) perforiert sind und sich durch die Applikation des Ballondrucks öffnen,
- und daß das über die Außenlumina (18) zugeführte bzw. eingefüllte Fluid mit Hilfe eines angelegten Drucks durch die Außenöffnungen (24) in das Gefäß bzw. die Gefäßwand applizierbar ist.

2. Manschettenkatheter nach Anspruch 1,
**dadurch gekennzeichnet,**
daß die Austrittsöffnungen (24) perforiert sind und sich durch die Applikation eines zusätzlichen Drucks auf die Außenlumina (18) öffnen.

3. Manschettenkatheter nach Anspruch 1,
**dadurch gekennzeichnet,**
daß auch auf der das zentrale Lumen (20) begrenzenden Innenwand zwischen den Außenlumina (18) in Längsrichtung verlaufende Radialeinschnitte (23) vorhanden sind, die bis zu einer ein Gelenk bildenden Restdicke verlaufen.

4. Manschettenkatheter nach einem oder mehreren der Ansprüche 1 bis 3,
**dadurch gekennzeichnet,**
daß die Perforation der Austrittsöffnungen (24) derart ausgeführt ist, daß sich die Austrittsöffnungen mit ansteigendem Druck vom distalen Ende ausgehend zum proximalen Ende des Kopfsegmentes (16) hin verlaufend öffnen.

5. Manschettenkatheter nach einem oder mehreren der Ansprüche 1 bis 4,
**dadurch gekennzeichnet,**
daß über den Umfang des Kopfsegmentes (16) zwischen sechs bis zwanzig Außenlumina (18) vorgesehen sind.

## Claims

1. A sleeve catheter for the wetting and infiltration of a vessel wall or other tubular organs with a fluid, in particular a medicament, having a head segment which is in the form of a sleeve, emits a medicament and as a result of the inflation of a balloon (12) can be expanded to bear against the inner wall of the vessel, characterised in that
- the sleeve catheter (10) has a ring of outer lumina (18) which in cross-section are distributed around a central lumen (20) and which terminate in outlet openings (24) in the head segment (16),
- with its central lumen (20) the sleeve catheter (10) can be slipped onto a balloon catheter (11) and engages at least partially over the balloon (12),
- the head segment (16) is provided externally, between the outlet openings (24), with radial indentations (22) extending in the longitudinal direction in such manner that the residual thickness which is not cut through forms a joint,
- the outlet openings (24) are perforated and open as a result of the application of the balloon pressure,
- and the fluid supplied and introduced via the outer lumina (18) can be applied, with the aid of an applied pressure, through the outlet openings (24) into the vessel or vessel wall.

2. A sleeve catheter according to Claim 1, characterised in that the outlet openings (24) are perforated and open as a result of the application of an additional pressure to the outer lumina (18).

3. A sleeve catheter according to Claim 1, characterised in that radial indentations (23) running in the longitudinal direction between the outer lumina (18) are also provided on the inner wall which delimits the central lumen (20), which radial indentations (23) extend up to a residual thickness which forms a joint.

4. A sleeve catheter according to one or more of Claims 1 to 3, characterised in that the perforation of the outlet openings (24) is formed in such manner that with increasing pressure the outlet openings open from the distal end towards the proximal end of the head segment (16).

5. A sleeve catheter according to one or more of Claims 1 to 4, characterised in that between six and twenty outer lumina (18) are provided over the periphery of the head segment (16).

## Revendications

1. Cathéter à manchon pour mouiller une paroi de vaisseau ou d'autres organes tubulaires par un fluide, en particulier un médicament, et infiltrer ce fluide dans la paroi, comportant un segment de tête réalisé comme un manchon et délivrant un médicament, segment qui se laisse évaser par gonflage d'un ballonnet (12) jusqu'à son application contre la paroi interne du vaisseau,
**caractérisé en ce**
- que le cathéter à manchon (10) présente une couronne de lumières extérieures (18) réparties, en section droite, autour d'une lumière centrale (20) et se terminant dans le segment de tête (16) par des orifices de sortie (24),
- que le cathéter à manchon (10) peut être enfilé par sa lumière centrale (20) sur un cathéter à ballonnet (11) pour entourer au moins partiellement le ballonnet (12),
- que le côté extérieur du segment de tête (16) est pourvu, entre les orifices de sortie (24), d'entailles radiales (22), orientées en direction longitudinale, de manière que l'épaisseur résiduelle non sectionnée forme une articulation,
- que les orifices de sortie (24) sont perforés et s'ouvrent par l'application de la pression au ballonnet, et
- que le fluide amené ou introduit à travers les lumières extérieures (18) est introduisible à travers les orifices extérieurs (24) dans le vaisseau ou la paroi de vaisseau à l'aide d'une pression appliquée.

2. Cathéter à manchon selon la revendication 1, caractérisé en ce que les orifices de sortie (24) sont perforés et s'ouvrent par l'application d'une pression supplémentaire aux lumières extérieures (18).

3. Cathéter à manchon selon la revendication 1, caractérisé en ce que la paroi interne délimitant la lumière centrale (20) présente également, entre les lumières extérieures (18), des entailles radiales (23), orientées dans la direction longitudinale, qui laissent subsister une épaisseur résiduelle formant une articulation.

4. Cathéter à manchon selon une ou plusieurs des revendications 1 à 3, caractérisé en ce que la perforation des orifices de sortie (24) est réalisée de manière que ces orifices s'ouvrent progressivement à partir de l'extrémité distale vers l'extrémité proximale du segment de tête (16) à mesure que la pression augmente.

5. Cathéter à manchon selon une ou plusieurs des revendications 1 à 4, caractérisé en ce que le nombre de lumières extérieures (18) réparties sur le pourtour du segment de tête (16) est compris entre six et vingt.
